# EUROPEAN PATENT APPLICATION

(11) **EP 2 659 832 A1**
(43) Date of publication of application: **06.11.2013**
(21) Application number: 13397508.6
(22) Date of filing: 11.04.2013
(51) Int. Cl.: A61B 5/00, A61B 5/11

(54) **Capacitive sensor made of fabric for measuring human movements**

(30) Priority: 30.04.2012 FI 20125472; 30.04.2012 FI 20125473
(71) Applicant: Univisio Oy, 38800 Jämijärvi (FI)
(72) Inventor: Korhonen, Aki, 33720 Tampere (FI); Vanhala, Jukka, 33960 Pirkkala (FI)
(74) Representative: Tampereen Patenttitoimisto Oy

(57) **Abstract**

A system comprises a sensor (100) comprising a frame (110), a first (120), a second (121) and a third (122) conductive electrode arranged in and/or on the frame (110), in which sensor (100) each of the three electrodes (120, 121, 122) is electrically insulated from the other electrodes. Said frame (110) comprises textile or fabric, and said electrodes (120, 121, 122) are arranged in parallel in the frame (110). The system further comprises a measuring unit (460) configured to measure at least one capacitance of the sensor in such a way that the capacitance of the electrode (120, 121, 122) is measured with respect to at least one adjacent electrode (121, 122, 120). Furthermore, the use of the sensor and a method for measuring a capacitance by the presented sensor. Further, a method for measuring the movements of a human body.

## Description

### Field of the invention

The invention relates to a capacitive sensor made of fabric. With the sensor, it is possible to electrically detect the presence and the movement of a person close to the sensor. The sensor can be provided in, for example, a mattress, a mattress topper, or a bed sheet. The invention also relates to a method for measuring at least two capacitances. Further, the invention relates to a method for measuring the movement of a person by a capacitive sensor.

### Background of the invention

Recently, people are increasingly interested in well-being. Population ageing increases the need for monitoring close relatives and other people to be cared for. Interest in one's own well-being also increases the need for monitoring oneself, for example during sleep. Monitoring can be implemented, for example, by measuring the activity of a person, such as oneself or a close relative.

A person's activity can be measured by sensors. The sensors can operate, for example, capacitively. Sensors can be placed in various seats, beds or mattresses. For example, it is possible to measure a change in the capacitance of the sensors, which change is caused by e.g. the person's breathing, wherein such measurements can be used for obtaining data on the person's breathing.

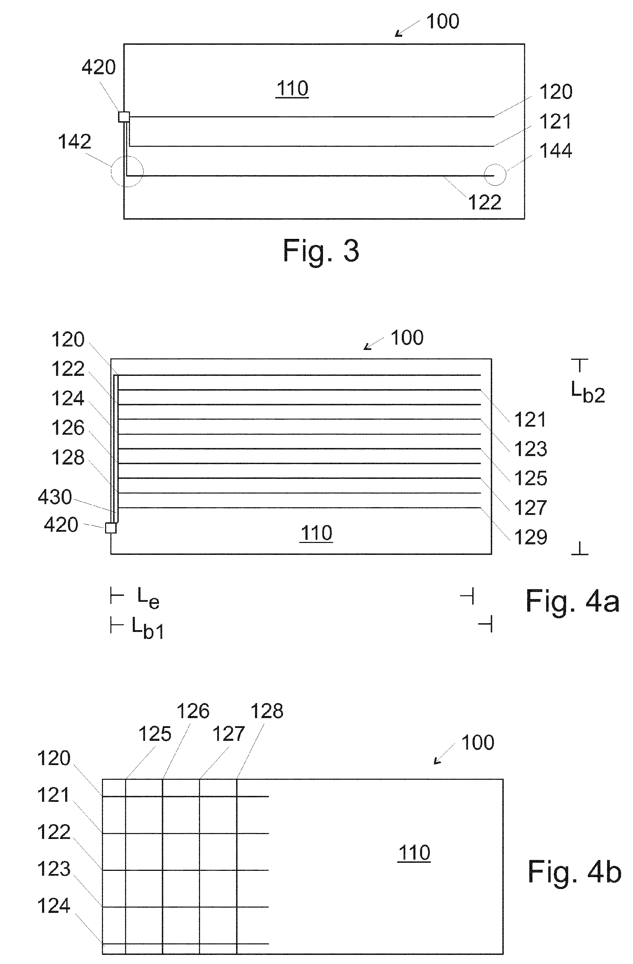

Document US 3,926,177 discloses a capacitive sensor which can be used for measuring a movement caused by a person's breathing while the person is sleeping or otherwise lying on a mattress. The mattress is equipped with a capacitive sensor. The operation of the sensor is based on two electrodes (Fig. 3 of the document; reference numerals 15 and 16), between which a resilient insulating layer is provided (Fig. 3 of the document; reference numeral 17), wherein a force exerted on the electrode causes compression of the insulation, which in turn is reflected as a change in the capacitance between the electrodes. The electrodes have a planar shape, and said insulation is provided between the planar electrodes, wherein the electrodes are on top of each other. Consequently, the sensor is used as a force or pressure sensor. The document also presents the shielding of these two electrodes with a ground plane (Fig. 3 of the document; reference numeral 14).

### Brief summary of the invention

It is an aim of the present invention to improve some drawbacks of the state of the art. The invention relates, on one hand, to a new measuring method for measuring at least two capacitances. The method can be used for measuring, for example, human movements. By the method, it is possible to utilize electrodes in measuring in a versatile way, whereby the measurement accuracy is improved with respect to the location on one hand and with respect to the capacitance on the other hand. Besides, the invention relates to a new sensor for measuring human movements. The new sensor is significantly less expensive than a sensor of prior art. Furthermore, the new sensor is easier to manufacture, wherein the manufacturing costs are lower than in the state of the art. Moreover, the new sensor is comfortable to use.

The sensor according to the invention is characterized in what is presented in the characterizing part of the independent claim 1.

The method according to the invention is characterized in what is presented in the characterizing part of the independent claim 10.

### Description of the drawings

In the following, the invention will be described in more detail with reference to the appended drawings, in which:
- Fig. 1: shows a sensor according to an embodiment, seen from above,
- Fig. 2a: shows a sensor according to Fig. 1, in the cross-sectional plane II-II of Fig. 1,
- Fig. 2b: shows the use of a sensor according to Fig. 1 for measuring the presence and location of a person, in the cross-sectional plane II-II of Fig. 1,
- Fig. 3: shows a sensor according to an embodiment, seen from above,
- Fig. 4a: shows a sensor according to an embodiment, seen from above,
- Fig. 4b: shows a sensor according to an embodiment, seen from above,
- Figs. 5a to 5c: show the use of a sensor according to an embodiment for measuring the presence and the location of a person, seen from the end of the sensor,
- Figs. 6a to 6c: show a method for measuring three capacitances of a sensor according to an embodiment,
- Figs. 7a to 7c: show a method for measuring three capacitances of a sensor according to an embodiment,
- Figs. 8a to 8c: show a method for measuring three capacitances of a sensor according to an embodiment,
- Figs. 9a and 9b: show a method for measuring the capacitance of a sensor according to an embodiment,
- Fig. 10: illustrates a principle of a circuit diagram for measuring the capacitance, and
- Fig. 11: shows a sensor according to an embodiment, seen from above, the sensor comprising a measuring unit.

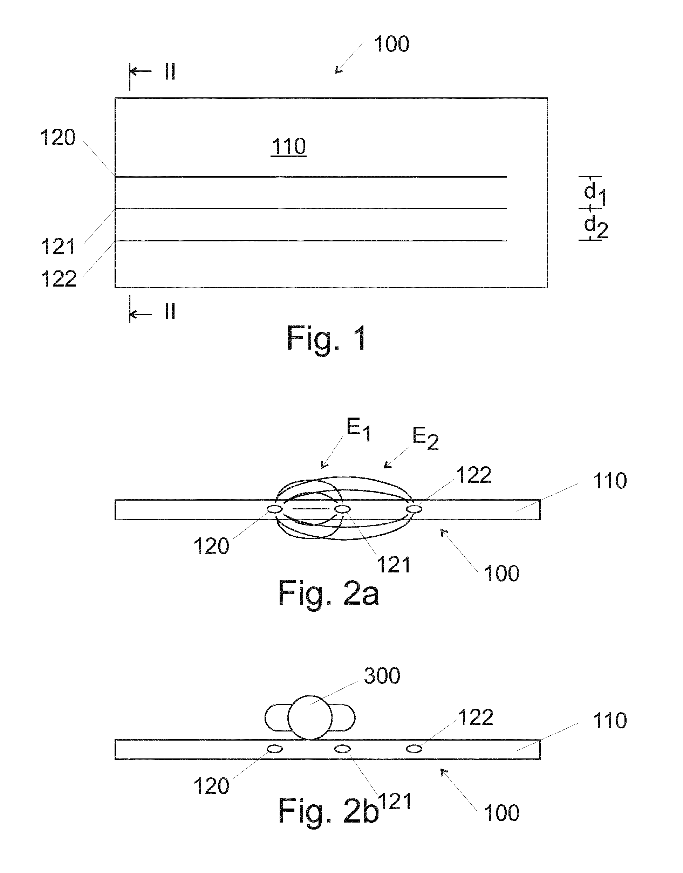

In Figs. 1 to 11, corresponding numerals or symbols refer to corresponding parts.

### Detailed description of the invention

Figure 1 shows a sensor 100 according to an embodiment of the invention, seen from above. The sensor 100 comprises a frame 110. The sensor 100 comprises a first conductive electrode 120, a second conductive electrode 121, and a third conductive electrode 122. The conductive electrode comprises electrically conductive material. By means of two electrodes, it is possible to measure the presence of a piece, such as a person. By means of three electrodes, it is also possible to measure the location of the person, because three electrodes can be used to measure if the person is particularly in the vicinity of the area between the first and second electrodes or particularly in the vicinity of the area between the second and third electrodes.

The first electrode 120 may be arranged in the frame 110, that is, inside the frame 110. The first electrode 120 may be arranged on the frame 110, that is, on either side of the frame 110. The first electrode 120 may be arranged partly in the frame 110 and partly on the frame 110. Thus, the first electrode 120 is arranged in the frame 110, on the frame 110, or both in the frame 110 and on the frame 110. Also, the second electrode 121 is arranged in the frame 110, on the frame 110, or both in the frame 110 and on the frame 110. Further, the third electrode 122 is arranged in a similar way in the frame 110, on the frame 110, or both in the frame 110 and on the frame 110.

Said electrodes 120, 121, 122 can have an elongated shape. Thus, their length is clearly greater than their width or thickness. The length may be, for example, at least tenfold with respect to the width or the thickness. Such an elongated conductor is easy to install in the frame of the sensor. Furthermore, the material costs of the elongated electrode are low. The dimensions of electrodes will be discussed in more detail later. The electrodes may also have other shapes. However, an electrical conductor extends from them, by means of which the capacitance of said electrode can be measured. The elongated electrodes have a longitudinal direction. Similarly, the electrical conductor connected to the electrode has a longitudinal direction.

Said electrodes 120, 121, 122 are aligned in parallel in or on the frame 110. In Fig. 2a, the electrodes 120, 121 and 122 are on the same plane, and furthermore, they are in the plane of the frame 110, wherein they are in parallel. The electrodes are not necessarily precisely in the same plane. In an example, the frame is planar and has a thickness. In this case, the electrodes may be configured in parallel in the plane of the frame, and at different heights in the thickness direction transverse to the plane of the frame. The positions for placing the electrodes will be discussed in more detail later. Even in this case, the electrodes are parallel. If the frame 110 is planar, the electrodes are next to each other, for example when a space is left between the electrodes in a direction parallel with the plane of the frame. Said direction of the plane of the frame may also be transverse to said longitudinal direction of the electrodes. If the electrodes have no longitudinal direction, the electrodes are parallel, for example, when a space is left between the electrodes in a direction that is parallel with the plane of the frame and transverse to the longitudinal direction of the electrical conductors connected to the electrodes. On the other hand, if the frame is not planar, the electrodes are in parallel, for example when a space is left between the electrodes in a direction transverse to the longitudinal direction of the electrodes. Furthermore, the electrodes may be parallel when a space is left between the electrodes in a direction that is transverse to the longitudinal direction of the electrical conductors connected to the electrodes.

The frame 110 may comprise electrically insulating material. Electrically insulating material refers to a material whose resistivity is at least 10⁹Ωm. Electrically conductive material refers to a material whose resistivity is at most 1Ωm. Resistivity ρ represents the specific resistance of a substance; and the resistance of a piece having a length L and a cross-sectional area A is pL/A. Electrical conductivity is inversely proportional to resistivity. It is also possible that the frame is at least weakly electroconductive. Thus, the sensor 100 also comprises an insulating layer on the electrodes. The insulating layer may be, for example, the sheath of an electric wire used as an electrode.

The first 120, second 121 and third 122 electrodes comprise electrically conductive material. In the application, no high current passes through the electrode (120, 121, 122) of the sensor 100, so that the electrical conductivity of the electrode (120, 121, 122) does not need to be particularly good; in other words, the resistivity does not need to be particularly low.

The first electrode 120 and the second electrode 121 are spaced from each other by a distance. The distance may depend on the point at which the distance is looked at. In the sensor 100 shown in Fig. 1, the distance between the first electrode 120 and the second electrode 121 is substantially constant, and it is denoted with the symbol d₁. The first electrode 120 is electrically insulated from the other electrodes. Thus, a first capacitance of the sensor is formed between the first electrode 120 and at least one other electrode. In a known way, the capacitance C between two electrodes depends, among other things, on the distance d₁ between the conductors and on the permittivity ε of the medium between the conductors. As the distance d₁ increases, the capacitance C decreases, and as the permittivity ε of the medium increases, the capacitance C increases.

The second electrode 121 and the third electrode 122 are spaced from each other by a distance. The distance may depend on the point at which the distance is looked at. For example, the electrodes are not necessarily straight in their longitudinal direction. In the sensor 100 shown in Fig. 1, the distance between the second electrode 121 and the third electrode 122 is substantially constant, and it is denoted with the symbol d₂. The second electrode 121 is electrically insulated from the other electrodes. Thus, a second capacitance of the sensor is formed between the second electrode 121 and at least one other electrode. Further, in the same way, a third capacitance of the sensor is formed between the third electrode 122 and at least one other electrode.

The first, second, and/or third capacitance of the sensor can be measured in a way to be described later. From the values of the capacitances it can be concluded, in the vicinity of which electrodes the person or a part of the person is. From the changes in the capacitances, it is possible to make conclusions on the movement and activity of the person.

Permittivity is often expressed as the product of absolute permittivity ε₀ and relative permittivity εᵣ. Absolute permittivity is also called vacuum permittivity and electric constant. Its numerical value is ε₀= 8,854×10⁻¹² F/m. Relative permittivity is for air about 1.0, for water about 81 and for cotton about 4 to 8. Permittivity is dependent on frequency. The above values have been measured at a low frequency, wherein the permittivity can also be called dielectric constant. Human tissue contains water about 40 to 80%, depending on the tissue in question. Thus, it is obvious that when a person approaches the sensor and part of the air is replaced with tissue that contains water, the permittivity is changed, whereby the capacitance is changed.

The sensor is advantageously used in applications for measuring the presence and movements of a person. More advantageously, the presence and movements of a person are measured in connection with normal functions. Such normal functions may include, for example, sleeping, sitting or other functions in a sitting or lying position. Thus, the measuring sensor is advantageously integrated in normal furniture or upholstery. In applications for measuring a person in a lying position, the sensor may be integrated in a mattress, a mattress cover, a mattress topper, or a cover for a mattress topper, or in bed linen, such as a bed sheet. In applications for measuring a person in a sitting position, the sensor may be integrated in a seat, seat upholstery, a seat cushion, or a seat cushion cover.

The sensor is easy to integrate in such applications when the frame 110 of the sensor 100 is made of textile or fabric. Such a frame 110 comprises textile or fabric. In some embodiments of the invention, the frame 110 comprises textile or fabric. The textile or fabric is a planar structure made of either threads or fibres. Textiles or fabrics made of threads include woven fabrics, knitted fabrics, braids, tulle fabrics, laces, and mesh structures, i.e. nettings. Fabrics made of fibres include non-woven fabrics and felts.

Materials for manufacturing textiles and fabrics include natural fibres of plant and animal origin, synthetic fibres, or blends of natural fibres and synthetic fibres. Natural fibres include, for example, cotton, flax, hemp, wool, and silk. Synthetic fibres include, for example, acryl, polyester and polyamide. According to the use, the frame 110 of fabric can be subjected to various finishing treatments to improve, for example, their waterproofness, windproofness, soil repellency, heat and fire resistance, and other desired properties. The electrode 120, 121, 122 may be, for example, an electric wire made of metal; an electrode sewn in the frame by conductive thread; an electrode printed on the frame with conductive ink or elastomer, such as silicone; or a band formed of conductive fabric.

The sensor 100 may comprise a separate electrically insulating layer, such as the sheath of a conductor, for insulating the electrode. The electrode may extend in one direction (longitudinal direction) and be substantially narrow in a direction (cross direction) transverse to this direction. In the longitudinal direction, the length of the conductor may be, for example, at least 30 cm, or more advantageously at least 80 cm, as will be presented later in connection with some embodiments. In its cross direction, the electrode may have, for example, a circular cross section. The dimension of the electrode in its cross direction may thus be, for example, 0.1 mm to 2 mm. The electrode may also be band-like, wherein it has two dimensions in its cross direction. The smaller dimension may be, for example, 0.1 mm to 2 mm. The greater dimension may be, for example, 0.2 mm to 30 mm. If the frame of the sensor is planar, the greater dimension may be provided, for example, in the plane of the frame of the sensor.

In an embodiment, the frame 110 of the sensor 100 comprises fabric, and the electrodes 120, 121, 122 are integrated (for example, attached, sewn, woven, inserted, or printed, such as embossed, with a conductive material) in the frame 110. Such a sensor does not comprise a separate base which would comprise said electrodes, but the frame 110 made of fabric or textile is used as such a base. Thus, the sensor can be used like a corresponding fabric with no integrated electrodes. Such a sensor is, among other things, flexible, rollable and foldable. Furthermore, it has a pleasant texture. Moreover, the sensor can be washable.

Figure 2a shows the sensor 100 in the cross-sectional plane II-II of Fig. 1. When the first electrode 120 is connected to a first electric potential, and both the second electrode 121 and the third electrode 122 are connected to a second electric potential, first of all, a collective electrode consisting of the second and third electrodes is formed. Furthermore, electric fields E₁ and E₂ are formed between the electrodes, as illustrated with corresponding lines in Fig. 2a. In the above described manner, the permittivity of the medium between the electrodes affects the capacitance between the electrodes. The medium between the electrodes refers to a medium, through which an electric field (electric fields E₁ and E₂) passes. Thus, the permittivity of the medium depends, not only on the medium which is directly between the electrodes, but also on the medium which is in the vicinity of the electrodes.

For example, Fig. 2b illustrates a situation in which a person 300 is in the vicinity of electrodes 120 and 121. In the situation of Fig. 2b, the above-mentioned first capacitance of the sensor 100 differs from the first capacitance of the sensor 100 of Fig. 2a, because the person 300 changes the permittivity of the medium. In Fig. 2b, the electrodes are arranged in a plane. Thus, the capacitance of the sensor is arranged to change in such a way that the distance between the electrodes remains substantially constant. The electrodes may be arranged in parallel at different height levels in the frame 110. Thus, the distance between the electrodes may slightly change when the person lies down on the frame 110.

The first capacitance of the sensor refers to the capacitance between the first electrode 120 and at least one other electrode. In a corresponding manner, the second capacitance of the sensor refers to the capacitance between the second electrode 121 and at least one other electrode. In a corresponding manner, the third capacitance of the sensor refers to the capacitance between the third electrode 122 and at least one other electrode. Thus, the first capacitance of the sensor may be formed between the first electrode and the electrically connected second and third electrodes. Alternatively, the first capacitance of the sensor may be formed between the first electrode and the second electrode when the third electrode is not connected electrically to the first or second electrode. In yet another alternative, the first capacitance of the sensor may be formed between the first electrode and the third electrode when the second electrode is not connected electrically to the first or third electrode. Corresponding observations apply to the second and third capacitance of the sensor. In the situation of Fig. 2b, the third capacitance of the sensor 100 does not necessarily differ from the third capacitance of the sensor 100 according to Fig. 2a, because the person 300 does not significantly change the permittivity of the medium in the vicinity of the third electrode 122.

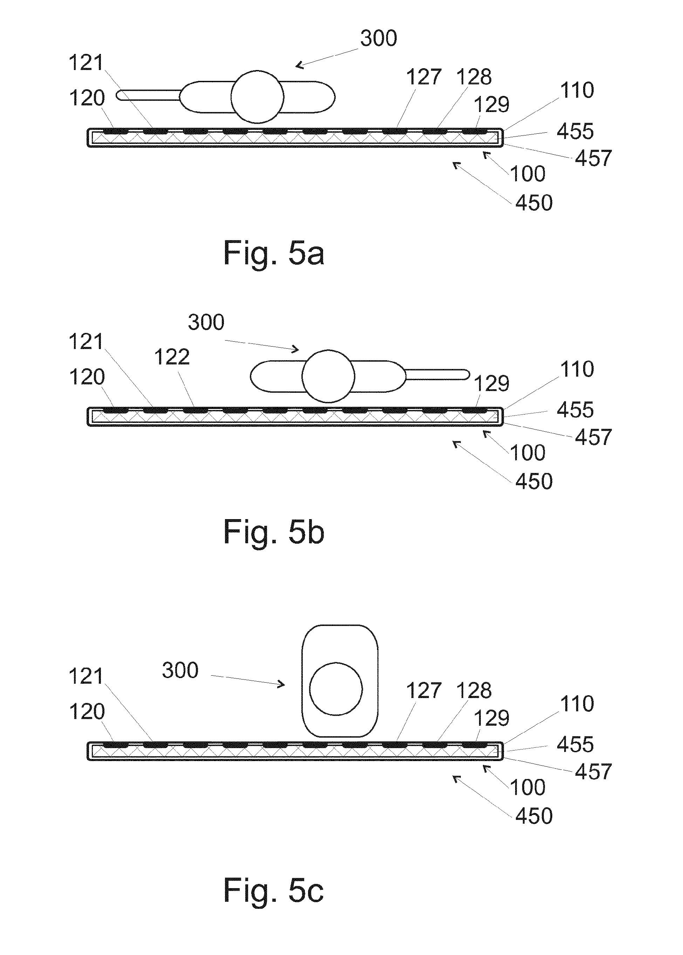

The frame 110 of the sensor 100 is advantageously planar. The first electrode 120 may be placed in the plane of the frame 110. The second electrode 121 and the third electrode 122 may also be placed in the plane of the frame 110. The electrodes may be placed inside the frame 110, *i.e.* in the frame 110, as in Fig. 2a, or they may be placed on the surface of the frame 110, that is, on the frame 110, as in Fig. 5a. The electrodes 120, 121, 122 may all be on the same surface of the frame, or one of them may be on a different surface than the other two. In the case of Fig. 5a, the frame may be, for example, a bed sheet, on whose surface the electrodes are embossed. The electrodes may be embossed, for example, on the lower surface of the bed sheet. The lower surface refers to the direction of the bed sheet in use. Thus, the electrodes do not touch the person to be measured during the measuring, and the texture is pleasant.

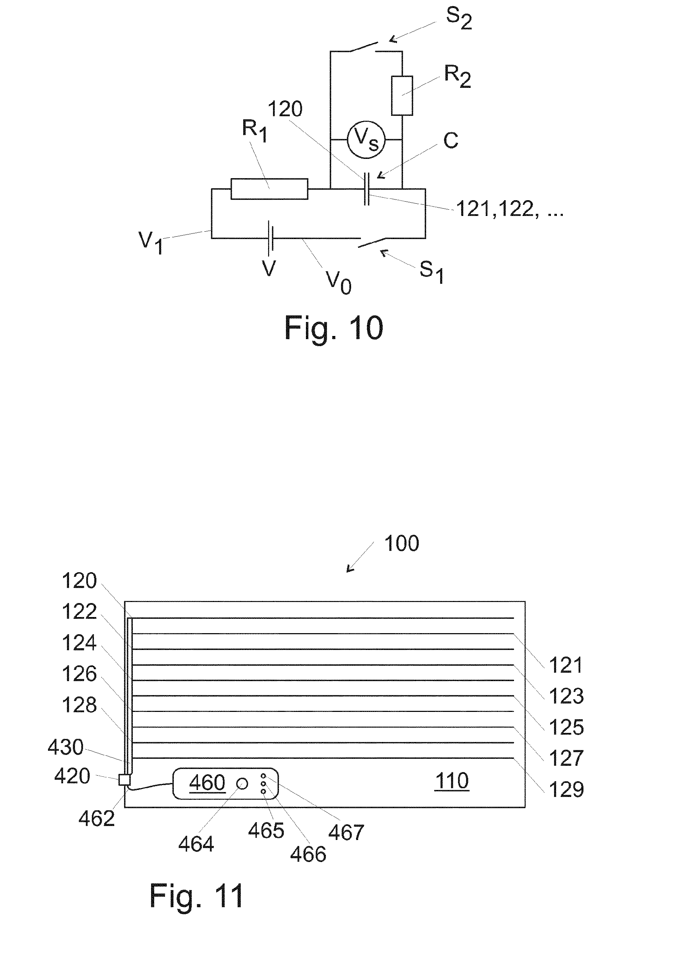

In Figs. 1 and 3, the first conductive electrode 120 is in the form of an extending conductor. In the figures, the second 121 and third 122 conductive electrodes are also in the form of extending conductors. In the figure, the conductor is drawn as a straight line, but the conductor may also run along a winding path. The electrodes may also have other shapes. The distance between the electrodes may be, for example, from 0.5 to 25 cm. Thus, particularly the distance between a conductive electrode and the closest other conductive electrode is within said range. In an embodiment, the distance between each conductive electrode and the closest other conductive electrode is within said range. With higher ranges, changes in capacitance are difficult to detect. The distance between the electrodes may not be greater than, for example, 8 cm, 12 cm, 18 cm, or 25 cm. On the other hand, with short distances, the number of electrodes needed in the sensor increases to a significant number. This increases, among other things, the manufacturing costs. The distance between the electrodes may be at least, for example, 0.5 cm, 3 cm, 7 cm, or 11 cm. Advantageously, the distance between electrodes is between 3 and 12 cm, preferably about 10 cm. The electrodes may be substantially parallel. The electrodes may also form an angle to each other. The electrodes can be considered to be substantially parallel, if the angle between straight lines drawn between the initial ends and the terminal ends of the electrodes is smaller than 10 degrees. In this context, the initial end refers to the point where the electrode departs from an edge of the sensor, as shown by reference numeral 142 in Fig. 3. The terminal end refers to the point where the electrode ends freely. In Fig. 3, the terminal end is denoted with the reference numeral 144. The electrode also has another end which terminates, for example, in a connector 420 (Fig. 3) or a measuring unit 460 (Fig. 11).

Advantageously, in the measuring area of the sensor, the distance between the electrodes is within the above-mentioned range. The sensor 100 shown in Fig. 3 further comprises a connector 420 for connecting electrodes 120, 121, 122 electrically to the rest of the system. Advantageously, the connector 420 comprises one contact for each electrode. The sensor 100 may also comprise several connectors. For example, one connector for each electrode.

The sensitivity of the sensor can be improved by the above described method in which at least two electrodes constitute a collective electrode of the sensor, and the remaining electrode forms the capacitance of the sensor with respect to said collective electrode. Thus, the surface area of the collective electrode is larger than the surface area of a single, electrically insulated electrode, which increases the sensitivity.

The location sensitivity of the sensor in one direction of the plane of the sensor can be improved by increasing the number of electrodes. Figure 4a illustrates such a situation. The sensor of Fig. 4a comprises ten electrodes 120, 121, 122, 123, ..., 129. The electrodes are in the form of continuous conductors. In particular, the length of the conductor is considerably greater than the thickness or width of the conductor. In the sensor 100, the electrodes 120 to 129 are advantageously placed in such a way that the longitudinal direction of the conductor is substantially parallel with the longitudinal direction of the person lying on the sensor. More generally, the longitudinal direction of the conductor of the sensor is advantageously substantially transverse to the assumed direction of movement of the person. The electrodes 120 to 129 are not necessarily parallel with the edge of any sensor. The planar sensor may have a rectangular, circular or other shape. The sensor is not necessarily planar. The sensor may also comprise another number of electrodes. The sensor may comprise several electrodes.

The location sensitivity of the sensor can also be improved by the above described method in which each electrode can be used as an electrode forming the capacitance of the sensor with respect to at least one other electrode. Thus, each electrode can be used for measuring the location. Furthermore, in the method, the capacitance of each electrode is measured with respect to at least one other electrode. Another alternative could be, for example, a solution in which every other electrode would act as a ground electrode. Thus, the location sensitivity would be only about half of what can be achieved in the above described way. In the presented method, a fixed ground electrode is not necessary either, but one or some of the electrodes are used in turn as a ground electrode.

In an embodiment, a bed sheet is used as the frame 110 of the sensor. The bed sheet is made of fabric. The bed sheet can be made of, for example, natural fibres. Thus, the sensor 100 comprises said bed sheet. The sensor also comprises electrodes arranged in or on the bed sheet (in or on the frame 110). The frame 110 of such a sensor is planar. Naturally, the frame 110 can also be rolled or folded to another shape, for storage or transportation. In a use situation, however, the frame 110 is substantially planar. When a bed sheet is used as the frame, the dimension of the planar frame in the first direction L_{b1} of the plane (Fig. 4a) is, for example, between 80 and 250 cm. The dimension of the planar frame in the second direction L_{b2} transverse to the first direction (Fig. 4a) is, for example, between 60 and 250 cm. The size of the bed sheet is influenced by, among other things, the size of the bed used. For example, the size of a sheet for a baby cot may be, for example, 60x80 cm, whereas the size of a sheet for a double bed may be, for exam-250×250 cm. The electrodes may extend from one side of the frame to the opposite side of the frame. In the case of a bed sheet, a mattress or a mattress topper, the electrodes advantageously extend in at least the direction which is intended as the longitudinal direction for the person using the bed sheet, mattress, or mattress topper. If the dimensions of the frame (bed sheet, mattress topper or mattress) of the sensor deviate from each other in said first and second directions, this direction is usually parallel with the greater dimension of the dimensions of the frame. In the case of a bed sheet, a mattress, or a mattress topper, the movement of the person takes place primarily in a direction transverse to the longitudinal direction of the person.

If a mattress topper or a mattress is used as the frame of the sensor, the electrodes are advantageously arranged in the top part of the frame or on the frame. The electrodes are arranged in the top part of the frame when at least part of the electrodes are placed at least partly above half-way in the thickness direction of the frame. In this context, the top part refers to the direction of the mattress or mattress topper during use. More advantageously, the electrodes are arranged in the top part of the frame in such a way that all the electrodes are placed at least partly above half-way in the thickness direction of the frame. Most advantageously, the electrodes are arranged in the top part of the frame in such a way that all the electrodes are placed completely above half-way in the thickness direction of the frame. Placed in this way, the measuring sensitivity of the sensor is good. Even in this case, the mattress remains comfortable to use, because the frame 110 of the sensor 100 comprises textile or fabric. Advantageously, the electrodes are flexible, to improve the comfort in use.

The length Lₑ of the electrodes may be the same as the dimension of the frame in the first or second direction. In the above described way, for example the first dimension of the bed sheet may be greater than the second one. Advantageously, the electrodes extend in parallel with the greater dimension of the dimensions of the frame, that is, in the direction of the first dimension of the frame. Thus, the length Lₑ of the electrodes may be the same as the dimension of the frame in the first direction. In Fig. 4a, the length of the electrodes extends in the first direction of the frame. The electrodes may also be slightly shorter, as shown in Fig. 4a. Advantageously, the length of the electrodes is at least in the order of the length of the upper body of an average person. For example, the length of the electrodes may be at least 30 cm. Advantageously, the length of the electrodes may be at least 80 cm. In relation to the dimension of the frame 110, the length of the electrodes may be at least about one third of the corresponding dimension of the frame. Said dimension of the frame may be the greater dimension of the frame, wherein the length of the electrodes extends in the longitudinal direction of the person using the sensor, such as a bed sheet. Thus, the length of the electrode is advantageously greater than one third of the greater dimension of the frame; that is, Lₑ>(1/3)max(L_{b1},L_{b2}). The electrodes does not necessarily extend all the way to the edge.

Figure 4a also shows conductors 430, by which a connector 420 is electrically connected to the electrodes 120 to 129. As will be presented later, in one embodiment each electrode 120, 121, 122, ..., 129 can be connected to both a first electric potential V₁ and a second electric potential V₂ in an alternating manner. Such a connection can be made by control electronics electrically connected to said connector 420.

As stated above, the sensor 100 advantageously comprises electrodes whose longitudinal direction is substantially transverse to the expected direction of movement of the person. The electrodes can extend substantially in the same direction, or at an angle to each other. If the direction of movement of the person is not known, it is possible to provide the sensor with electrodes which are in a direction substantially transverse to an electrode. Even in such a case, the sensor comprises at least three electrodes extending in a first direction. In Fig. 4b, the sensor 100 comprises five electrodes 120 to 124 extending in the first direction. Further, the sensor comprises at least three electrodes extending in a second direction. In Fig. 4b, the sensor 100 comprises four electrodes 125 to 128 extending in the second direction. The second direction may be substantially transverse to the first direction. The second direction may also be at another angle to the first direction.

The frame 110 of the sensor can also be a mattress topper or a mattress. Even in this case, the electrodes may be arranged in or on the frame. In the case of a mattress topper, the electrodes are advantageously arranged in the lining protecting the resilient filling of the mattress topper, on the lining, or between the actual resilient filling of and the lining of the mattress topper, inside the lining. In a corresponding manner, in the case of a mattress, the electrodes are advantageously arranged in, on or under the lining protecting the mattress. More generally, the frame of the sensor may be bed linen, such as a bed sheet, a blanket cover, or a pillow case. The electrodes may be arranged in the top part of the frame 110 or on the frame 110, in the thickness direction of the frame, wherein the measuring sensitivity of the sensor is good. The thickness of the sensor depends on the implementation of the sensor. Typically, the thickness of a mattress is less than 50 cm, for example from 5 to 40 cm, or for example from 10 to 35 cm. Typically, the thickness of a mattress topper is less than 10 cm, for example from 3 to 8 cm. Typically, the thickness of a bed sheet is small, less than 2 mm.

The frame 110 of the sensor may also be upholstery fabric more generally. Even in this case, the electrodes may be arranged in or on the frame. The electrodes may be arranged in upholstery, that is, inside the upholstery fabric, on the outer surface of the upholstery fabric, or on the inner surface of the upholstery fabric. Upholstery fabric can be used, for example, for upholstering a piece of furniture. Upholstery fabric can be used, for example, for upholstering a seat. Upholstery fabric can be used, for example, for upholstering a seat in a vehicle. A vehicle, such as an automobile, a train, a ship, or an aeroplane, may comprise a seat upholstered in this way.

One advantage of the sensor is its simple implementation. Due to the measuring method used, the sensor does not necessarily comprise a compressible core, by means of which the electrodes would move with respect to each other. Thus, the sensor is easier to make of elongated conductors. Moreover, the sensor does not necessarily comprise a separate protective ground plane. A simple solution is possible, because the sensor is not intended for measuring pressure variations but the movement and presence of a human body or body part. A simple structure is also possible because the movements to be measured are relatively large. Furthermore, the usability of the simple structure is enhanced by the fact that the electrodes can be used in a versatile way as both the ground plane and the measuring electrode. Moreover, the user experience of the simple structure is improved by the fact that the frame comprises textile or fabric. For example, the presented simple structure can be used for measuring the movements and presence of a person. However, for example small movements caused by the breathing of a person staying substantially still are not easy to detect by a sensor. Neither is it easy to detect movements caused by the heartbeat of a person.

Figures 5a to 5c illustrate the use of the sensor 100 for measuring the movements of a sleeping person 300. In Fig. 5a, a mattress topper 450 is used as the sensor 100. The mattress topper 450 comprises a resilient filling 455 and a protective lining 457. The lining 457 acts as the frame 110 for the sensor 100. The sensor 100 also comprises conductive electrodes 120, 121, 127, 128, and 129 provided on the frame 110. For clarity, the third 122, fourth 123, fifth 124, sixth 125, and seventh 126 electrodes are not denoted with reference numerals in Figs. 5a and 5c. The third electrode 122 is denoted with a reference numeral in Fig. 5b.

In Figs. 5a to 5c, the person 300 to be measured is in different locations with respect to the sensor 100 and/or in different positions with respect to the sensor 100. Because the permittivity of a human being is higher than that of air, in Fig. 5a particularly the first capacitance of the sensor is higher than in a situation in which the person 300 is not in the vicinity of the first electrode 120 of the sensor 100 (Figs. 5b and 5c). In a corresponding manner, in the case of Fig. 5b, the tenth capacitance of the sensor (*i.e.* the capacitance of the electrode 129 with respect to at least one other electrode) is higher than in the case of Fig. 5a. Also, the position, and a particularly a change in the position, of an arm or a leg can be measured by means of changes in the capacitance. For example, said tenth capacitance of the sensor is higher in the case of Fig. 5b than Fig. 5c.

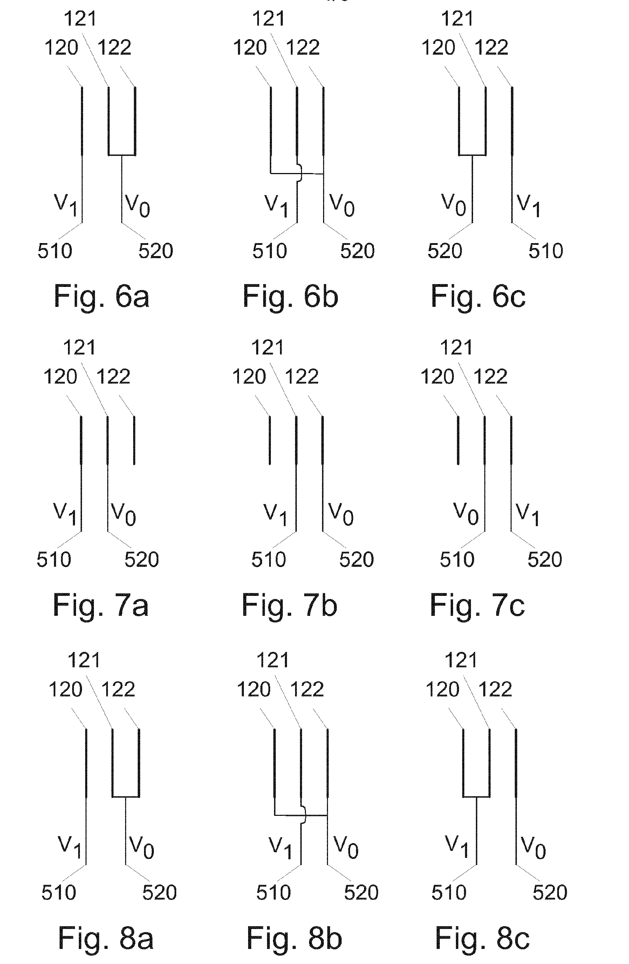

One capacitance of the sensor can be measured, for example, by connecting one electrode to the first potential and at least one other electrode to the second potential. In an embodiment of the invention, one electrode is connected to the first potential and one other electrode to the second potential, for measuring the capacitance of the sensor. In an embodiment of the invention, one electrode is connected to the first potential and one other electrode to the second potential, where said one other electrode is an electrode adjacent to said one electrode, for measuring the capacitance of the sensor. Advantageously, one capacitance of the sensor can be measured by connecting one electrode to the first potential and at least two other electrodes collectively to the second potential. More advantageously, said two other electrodes are electrodes adjacent to said one electrode; in other words, said one electrode is left between said two other electrodes. Most advantageously, one capacitance of the sensor can be measured by connecting one electrode to the first potential and all the other electrodes collectively to the second potential. The second potential can be, for example, the ground plane. The electrodes connected to the second potential constitute a collective electrode of the sensor. The capacitances of the sensor can be measured, for example, by connecting one electrode to the first potential and the other ones collectively to the second potential, in an alternating manner to be described below. The capacitance can also be measured by means of alternating current or voltage, whereby the electrodes are not connected to a potential that remains constant in time. Figures 6a to 6c show a principle of measuring the capacitances of the sensor, when the sensor comprises three electrodes 120,121 and 122.

In the measuring method shown in the drawings, each electrode can act both as a measuring electrode and as a ground electrode. For example, electrode number N can act as a measuring electrode when the capacitance number N of the sensor is measured, the other electrodes acting as a collective electrode (for example, as the ground plane). It is also possible that the electrode number N acts as the measuring electrode when the capacitance number N of the sensor is measured, and only one other electrode acts as the ground plane. In a corresponding manner, when measuring another capacitance of the sensor than the capacitance number N, the electrode number N can act as the ground plane or as a part of a collective electrode (the ground plane). This simplifies the structure of the sensor, because there is no need for one or more separate ground electrodes. Combining several electrodes to form a collective second electrode, for example the ground plane, increases the capacitance of the sensor and decreases the susceptibility of the sensor to external disturbances. In this way, the collective ground plane (or another plane of electric potential) improves the sensitivity of the sensor.

As shown in Fig. 6a, when measuring the first capacitance of the sensor,
- the second conductive electrode 121 and the third conductive electrode 122 of the sensor are electrically connected together, wherein the first collective electrode of the sensor is formed, and
- the first electrode 120 is electrically insulated from said first collective electrode of the sensor, wherein the first capacitance of the sensor is formed between the first conductive electrode 120 and the first collective electrode.

The first capacitance can be measured, for example, by connecting different electrodes to different potentials and by measuring the rise time of the voltage of the sensor in a way to be described later. In Fig. 6a, the first electrode 120 of the sensor is connected to the first potential V₁ and the other electrodes 121, 122 to the second potential V₀. The connection will be discussed in more detail later, among other things in the context of Fig. 10. The second potential can be, for example, the ground plane. Thus, the first capacitance of the sensor is formed between the first electrode 120 and the collective electrode formed by the other electrodes 121 and 122. The capacitance can also be measured from the oscillation frequency of an oscillating circuit in a way to be described later, wherein the sensor is supplied with an alternating voltage or an alternating current. Depending on the frequency of the oscillating voltage, particularly at a high frequency, the whole electrode is not necessarily in the same potential, but the potential varies in time and location.

As shown in Fig. 6b, when measuring the second capacitance of the sensor,
- the first conductive electrode 120 and the third conductive electrode 122 of the sensor are electrically connected together, wherein the second collective electrode of the sensor is formed, and
- the second electrode 121 of the sensor is electrically insulated from said second collective electrode of the sensor, wherein the second capacitance of the sensor is formed between the second conductive electrode 121 and the second collective electrode.

In Fig. 6b, the second electrode 121 of the sensor is connected to the first potential V₁ and the other electrodes 120, 122 to the second potential V₀. The second capacitance of the sensor is formed between the second electrode 121 and the collective electrode (for example, the ground plane) formed by the other electrodes 120 and 122.

As shown in Fig. 6c, when measuring the third capacitance of the sensor,
- the first conductive electrode 120 and the second conductive electrode 121 of the sensor are electrically connected together, wherein the third collective electrode of the sensor is formed, and
- the third electrode 122 of the sensor is electrically insulated from said third collective electrode of the sensor, wherein the third capacitance of the sensor is formed between the third conductive electrode 122 and the third collective electrode.

In Fig. 6c, the third electrode 122 of the sensor is connected to the first potential V₁ and the other electrodes 120, 121 to the second potential V₀. The third capacitance of the sensor is formed between the third electrode 122 and the ground plane formed by the other electrodes 120 and 121.

As shown in Fig. 7a, the first capacitance of the sensor could also be formed by
- electrically insulating the first electrode 120 from the other electrodes (121, 122) and by
- electrically insulating the second electrode 121 from the third electrode 122, wherein the first capacitance of the sensor is formed between the first conductive electrode 120 and another electrode (121, 122).

The capacitance can be measured in a way to be described below. In a corresponding manner, the other capacitances can also be formed in such a way that one electrode acts as the measuring electrode and only one other acts as the ground plane, as illustrated in Figs. 7b and 7c. When only one other electrode is used as the ground plane, one electrode is connected to the first potential and one other electrode is connected to the second potential. The other electrodes are insulated from said potentials.

Information about the movements of a person is obtained, for example, by measuring only the first and third capacitances of the sensor. Advantageously, the second capacitance of the sensor is measured as well.

In the embodiment shown in Figs. 6a to 6c, the first 120, second 122, and third 122 conductive electrodes can each be connected to both the first and the second electric potential in an alternating manner. In a corresponding manner, each electrode can be electrically connected to each of the other electrodes to form a collective electrode. If the sensor comprises three electrodes, each electrode can be electrically connected to both of the other electrodes, one at a time. As shown in Figs. 6 and 8, one electrode at a time forms the capacitance of the sensor and is thus electrically insulated from the other electrodes. In a corresponding manner, in Figs. 6 and 8, each electrode can be electrically insulated from all the other electrodes. If the sensor comprises several electrodes, each electrode can be electrically connected to at least one other electrode, to form a collective electrode of the sensor. Advantageously, each electrode can be electrically connected to several other electrodes to form a collective electrode of the sensor. Two electrodes are electrically connectable, if a connection with a low electrical resistance, for example lower than 10 Ω, can be formed between them. Typically, the electrical connection can be implemented with conductors, wherein the resistance is considerably lower than this. An electrode can be electrically insulated, if said electrical connection can be switched off. If the electrical connection has been switched off, the resistance of the electrode with respect to the other electrodes is high, for example higher than 100 Ω, because the frame 110 of the sensor is advantageously made of an electrically insulating material, and the electrode itself may comprise an insulating layer. Typically, if the electrical connection has been switched off, the resistance of the electrode with respect to the other electrodes is considerably higher than this.

Figures 8a to 8c show a measuring method that is slightly different from that shown in Figs. 6a to 6c. Figures 8a and 8b are identical with the Figs. 6a and 6b. As shown in Fig. 8c, when the third capacitance of the sensor is measured, the third electrode 122 of the sensor is connected to the second potential V₀ and the other electrodes 120, 121 to the first potential V₁. The third capacitance of the sensor is formed between the third electrode 122 and the voltage level formed by the other electrodes 120 and 121, the third electrode 122 of the sensor being in the electric ground plane.

In the embodiment shown in Figs. 8a to 8c, only the first 120 and the second 121 conductive electrodes can each be connected to both the first and the second electric potential, one at a time. The third electrode 122 is connected to the second potential V₀ only.

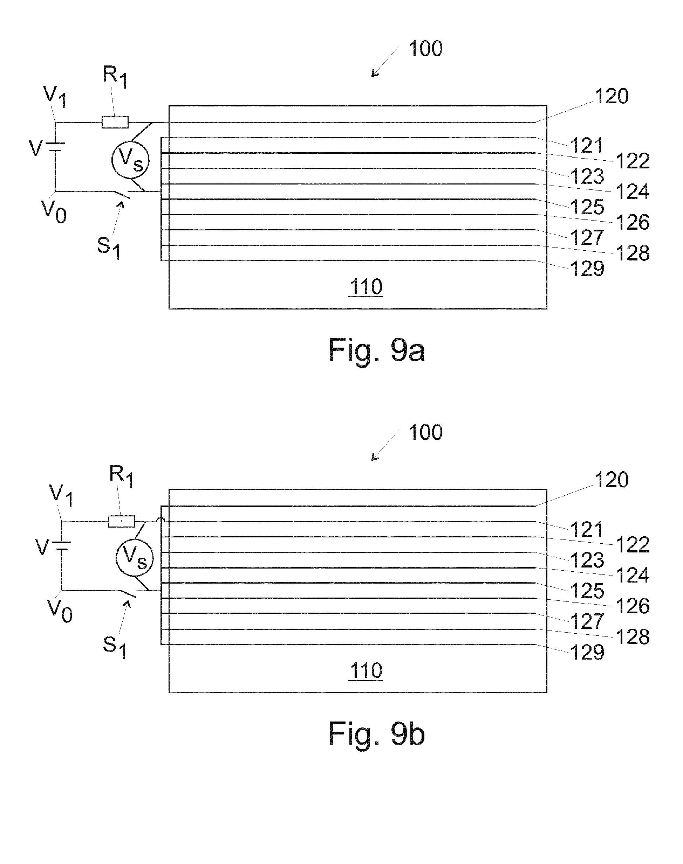

Further, Fig. 9a illustrates the measurement of the first capacitance of the sensor when the sensor comprises ten electrodes. In Fig. 9a, when the first capacitance of the sensor is measured, the first electrode 120 of the sensor is connected to the first potential V₁ and the other electrodes 121, 122, ..., 129 to the second potential V₀. Said other electrodes 121 to 129 thus form the first collective electrode of the sensor. The second potential V₀ can be, for example, the ground plane. Thus, the first capacitance of the sensor is formed between the first electrode 120 and the ground plane formed by the other electrodes 121 to 129. The figure also shows a front resistor R₁ for the charge of the first capacitance of the sensor, via which the electrode to be measured is connected to the first potential. Furthermore, a voltage source is shown, across which a voltage difference V prevails. Thus, the first potential is exactly by the voltage V higher than the second potential: V₁=V₀+V. The current circuit can be closed by a switch S₁, wherein the capacitance of the sensor is charged. Figure 9a further shows a voltmeter for measuring the voltage Vₛ across the first capacitance of the sensor. Other components possibly used in the measurement are shown in more detail in Fig. 10.

Further, Fig. 9b illustrates the measurement of the second capacitance of the sensor according to Fig. 9a. According to Fig. 9b, when the second capacitance of the sensor is measured, the second electrode 121 of the sensor is connected to the first potential V₁ and the other electrodes 120, 122, 123, 124..., 129 to the second potential V₀. Said other electrodes 120, 122 to 129 thus form the second collective electrode for the sensor. The second potential can be, for example, the ground plane. Thus, the second capacitance of the sensor is formed between the second electrode 121 and the ground plane formed by the other electrodes 120 and 122 to 129. Figure 9b further shows a voltmeter for measuring the voltage Vₛ across the second capacitance of the sensor. In an embodiment, the collective electrode is formed by connecting only two electrodes electrically together. With reference to Fig. 9b, the second collective electrode of the sensor can be formed, for example, by the electrodes 120 and 122 closest to the second electrode 121 of the sensor.

The capacitance of the sensor can be measured by known techniques. The capacitance can be determined, for example, from the voltage rise time across the sensor, when the sensor is charged with a known voltage by using a known front resistor. Such a circuit is shown in Figs. 9a, 9b and 10. Figures 9a, 9b and 10 show a voltage source generating a voltage V across itself. A voltmeter measures the voltage Vₛ across a capacitor, the capacitance of the capacitor being indicated with the letter C. It is obvious that the capacitance C of the capacitor is exactly a capacitance of the sensor 100 as described above. To emphasize this, the first electrode of the capacitor is, in the figure, marked as the first electrode 120 of the sensor, and the second electrode of the capacitor is marked as the other electrodes 121, 122, ... of the sensor. In the above described way, said other electrodes comprise at least one other electrode of the sensor, for example the second 121 or third 122 electrode. Advantageously, a collective electrode is formed of other electrodes of the sensor, wherein said other electrodes comprise two or more electrodes.

By means of the switch S₂ shown in Fig. 10, the capacitor can be uncharged through a dumping resistor R₂. The capacitor can be charged by means of the voltage source, if the voltage across the capacitor is higher than the voltage of the voltage source and if the switch S₁ is closed and the switch S₂ is open. Under certain conditions, charging is also possible when the switch S₂ is closed. When an empty capacitor is charged, the voltage of the capacitor rises in a known way towards the voltage of the voltage source in such a way that the voltage difference is reduced exponentially with respect to time. In a known manner, Vₛ=V₀(1-exp(-t/R₁C)), where t is time and R₁ is the front charging resistor (Fig. 10). By measuring the voltage Vₛ as a function of time, the capacitance C can be determined from said equation.

In a known manner, the capacitance can also be determined from the oscillation frequency of a series or parallel resonance circuit. In a series resonance circuit, an inductance (a coil) is connected in series with the capacitance of the sensor. The oscillation frequency of the series resonance circuit is inversely proportional to the square root of the product of the inductance and the capacitance. In a parallel resonance circuit, an inductance (a coil) is connected in parallel with the capacitance of the sensor. The oscillation frequency of the series resonance circuit is also inversely proportional to the square root of the product of the inductance and the capacitance. Possible resistances in the circuit affect the oscillation frequency.

It is obvious that the same methods can be used for measuring several capacitances of the sensor, for example for measuring each capacitance of the sensor 100.

In an example, a resistor having a value of R₁ = 10 MΩ was used as the front charging resistor R₁. The voltage of the voltage source was 3 V. The sensor was provided in a mattress topper, and the electrodes were about 180 cm long conductors having a substantially circular cross-section and a cross-sectional diameter of about 0.5 mm. The mattress topper was provided with 7 electrodes with a spacing of about 10 cm. The capacitances of the sensor were measured from the voltage rise time of the capacitance. The rise time was determined as the time in which the voltage across the capacitance rose to a range of approximately 60 to 70% of the voltage of the voltage source. Thus, the measuring time for each capacitance was about 250 µs when no person was lying on the mattress topper (low capacitance). The time for measuring the capacitance was about 350 to 450 µs when a person was lying on the mattress topper (high capacitance). For improving the measurement accuracy, each capacitance of the sensor was measured several times, and the average of these was given as the measurement result. As the dumping resistance R₂, R₂ = 10 kΩ was selected. Thus, discharging the capacitor took about one thousandth of its charging time.

With reference to Fig. 11, the sensor 100 (such as a mattress topper) advantageously comprises a measuring unit 460 as well. The measuring unit 460 comprises measuring electronics, and the measuring unit 460 is configured to measure the capacitances of the sensor. The measuring unit 460 is electrically connected to the rest of the sensor 100 by a cable 462 which may comprise several individual conductors, for example one conductor for each electrode 120 to 129. The cable 462 may be connected to the sensor, for example, by means of a connector 420, or the cable 462 may be connected directly to the electrodes. It is also possible that electrodes 120 to 129 extend directly to the measuring unit 460, in which case no separate cable 462 is needed.

The measuring unit 460 is configured to measure the capacitances of the sensor 100. In an embodiment, the measuring unit 460 is configured to measure the capacitance corresponding to each electrode of the sensor 100, wherein the number of capacitances of the sensor to be measured is equal to the number of electrodes. The measuring unit 460 is configured to measure the capacitances of the sensor 100, for example, in one of the above described ways. With reference to Figs. 6a to 6c, the measuring unit is, in an embodiment, configured
- to electrically connect the second conductive electrode 121 of the sensor and the third conductive electrode 122 of the sensor together,
- to electrically insulate the first electrode 120 of the sensor from the other electrodes of the sensor, whereby a first capacitance of the sensor is formed between the first conductive electrode 120 and the other electrodes,
- to measure said first capacitance,
- to electrically connect the first conductive electrode 120 of the sensor and the second conductive electrode 121 of the sensor together,
- to electrically insulate the third electrode 122 of the sensor from the other electrodes of the sensor, whereby a third capacitance of the sensor is formed between the third conductive electrode 122 and the other electrodes, and
- to measure said third capacitance.
The measuring unit 460 may be configured to measure the other capacitances of the sensor in a corresponding way. The measuring unit 460 may comprise a microcontroller configured to perform said electric connections and insulations.

With reference to Figs. 7a to 7c, the measuring unit is, in an embodiment, configured
- to electrically insulate the third electrode 122 of the sensor from the other electrodes of the sensor,
- to electrically insulate the first electrode 120 of the sensor from the other electrodes of the sensor, whereby a first capacitance of the sensor is formed between the first conductive electrode 120 and the second conductive electrode 121,
- to measure said first capacitance,
- to electrically insulate the first electrode 120 of the sensor from the other electrodes of the sensor,
- to electrically insulate the third electrode 122 of the sensor from the other electrodes of the sensor, whereby a third capacitance of the sensor is formed between the third conductive electrode 122 and the second conductive electrode 121, and
- to measure said third capacitance.

The measuring unit 460 may be configured to measure the other capacitances of the sensor in a corresponding way. The measuring unit 460 may comprise a microcontroller configured to perform said electric connections and insulations.

The measuring unit 460 may further be configured to perform at least one of the following:
- to display the measurement values of the capacitances of the sensor 100,
- to store the measurement values of the capacitances of the sensor 100 in a memory,
- to transmit the measurement values of the capacitances of the sensor 100 in a wired or wireless manner,
- to generate an activity value by means of the measurement values of the capacitances of the sensor 100,
- to display said activity value,
- to store said activity value, and
- to transmit said activity value in a wired or wireless manner.

The above mentioned memory can be, for example, a memory card. The memory card may be detachable from the measuring unit 460 for transferring the measurement values to, for example, a computer. The measurement values and/or the activity value can be transmitted to e.g. a computer, a mobile phone, a monitoring system, or a home control system. The monitoring system can store the data in, for example, a patient database. The home control system can start, on the basis of the data, other functions or devices, such as a coffeemaker, a television, or a computer.

In an embodiment, the measuring unit 460 is configured to generate an activity value by means of the measurement values of the capacitances of the sensor 100 and to display said activity value by turning on a light source corresponding to said activity value. For example, a light source 467 emitting green light (Fig. 11) can lit when the activity level has been low, wherein the person has presumably slept well. In a corresponding manner, a red light source 465 can be lit when the activity level has been high, wherein the person has presumably slept badly. An activity value between these can be displayed, for example, by switching on a light source 466 emitting yellow light. The measuring unit can be controlled, for example, by means of a push button 464.

It is obvious that the measuring unit 464 can also comprise other kinds of control devices, such as switches and several push buttons. It is also obvious that the measuring unit 464 may comprise a different kind of a display for displaying measurement values and/or the activity level to the user. The measuring unit 464 is powered by electricity. The required electric power can be supplied from, for example, one or more batteries, accumulators or powerful capacitors, or for example from a mains network, directly or via a transformer.

In the embodiments shown in the figures, the electrodes are numbered in an order according to their location. It is obvious that the electrodes can also be numbered in another way.

The presented measuring method can also be applied more generally for measuring capacitances by means of at least three electrodes. These three electrodes may be provided in a sensor that also comprises a frame, for example a frame comprising fabric. However, the measuring method can also be applied in a case in which at least three separate conductive electrodes are available.

With reference to Figs. 6a to 9b, at least one electrode can be used in the method, depending on the capacitance to be measured, either as a ground potential electrode or as a measuring potential electrode. In an embodiment of the method, said at least one electrode can also be used as a floating potential electrode. In this context, the floating potential electrode refers to an electrode which is (i) electrically insulated from the electrode (or electrodes) electrically connected to the ground potential and (ii) is electrically insulated from the electrode (or electrodes) connected to the electrically measuring potential.

With reference to Figs. 6a to 9b, one electrode can be electrically insulated from the other electrodes in the method. Furthermore, at least two of said other electrodes can be electrically connected together to form a collective electrode. Furthermore, the capacitance between said one electrode and said collective electrode can be determined.

With reference to Figs. 6a to 9b, one electrode can be electrically insulated from the other electrodes in the method. Furthermore, all the electrodes except for said one electrode can be electrically connected together to form a collective electrode. Furthermore, the capacitance between said one electrode and said collective electrode can be determined.

In an embodiment, at least two electrodes are electrically connected together, and the remaining third electrode is electrically insulated from these, wherein a capacitance of the structure is formed. This capacitance can be measured by a known technique. By changing the connection of the conductors, as stated in the context of Figs. 6 to 9, it is possible to measure the other capacitances of the structure. From the values of the measured capacitances, conclusions can be made on the movement of any piece, such as a human body, in the vicinity of the electrodes. The method is naturally suitable for use also in connection with the sensor structures presented in the context of Figs. 1 to 5. The method has the advantage that no separate ground electrode is needed, because at least one other electrode can be used as the ground electrode. Furthermore, the method has the advantage of more accurate location sensitivity, because the capacitances can be measured from each electrode instead of part of them functioning as a fixed ground plane. Yet another advantage of the method is higher sensitivity, because the collective electrode formed by several electrodes is larger in size than a single electrode, whereby the capacitance of such a sensor provided with a collective electrode is higher than that of a sensor in which only one pair of electrodes is used as a capacitance.

In an embodiment of the invention, four electrodes are used. In an embodiment of the invention, five electrodes are used. In an embodiment of the invention, six electrodes are used. As presented above, in some other embodiments, it is possible to use, for example, three, seven or ten electrodes.

In some embodiments, at least one electrode is electrically insulated from the other electrodes. Furthermore, at least two other electrodes are electrically connected to one sensor, for forming a collective electrode. Thus, the capacitance of the sensor is formed between said one electrode and said collective electrode of the sensor. Said capacitance of the sensor can be measured by a known technique. By changing the electrode to be electrically insulated from the other electrodes, it is possible to measure the capacitance of the sensor corresponding to each electrode with respect to the collective electrode formed by at least two other electrodes. Depending on the number of electrodes, the remaining electrodes are electrically insulated from said one electrode and said collective electrode.

In one embodiment, three electrodes are provided. Thus, one electrode can be connected to a first potential and two electrodes to a second potential, for measuring the capacitance. For example, the electrodes may be in parallel. When measuring the capacitance corresponding to the middle electrode of the sensor, the outermost ones can be connected together to form a collective electrode, and the middle electrode can be insulated from this collective electrode (Figs. 6b and 8b).

When measuring the capacitances corresponding to the outermost electrodes, a common electrode can be formed of the other electrodes (Figs. 6a, 6c, 8a, and 8c). Alternatively, when measuring the capacitances corresponding to the outermost electrodes, the capacitance of the outermost electrode can be measured with respect to one other electrode only. Thus, the capacitance can be measured, for example, with respect to the adjacent electrode (Figs. 7a and 7c). Advantageously, three capacitances are measured, one capacitance corresponding to each electrode.

In an embodiment, four electrodes are provided. Thus, one electrode can be connected to a first potential and two electrodes to a second potential. The remaining fourth electrode can be either connected to said second potential, to form a larger collective electrode, or electrically insulated from both the first and the second potential. The formed capacitance of the sensor can be measured. If the electrodes are placed in parallel, said one electrode advantageously remains between said two electrodes. At the edge of a row of electrodes, however, this is not possible. When measuring the capacitance corresponding to the outermost electrode, a collective electrode is not necessarily formed of several electrodes. The formed capacitances of the sensor can be measured by changing the one electrode connected to the first potential. If a collective electrode is formed of several electrodes by connecting them electrically together, said one electrode is advantageously left between two such electrodes which belong to the collective electrode. Advantageously, a total of four capacitances are measured, one capacitance corresponding to each electrode.

In an embodiment, five electrodes are provided. Thus, one electrode can be connected to a first potential and two electrodes to a second potential. The remaining fourth electrode can be either connected to said second potential, or electrically insulated from both the first and the second potential. The remaining fifth electrode can be either connected to said second potential, or electrically insulated from both the first and the second potential. The formed capacitance of the sensor can be measured. If the electrodes are placed in parallel, said one electrode advantageously remains between said two electrodes. At the edge of a row of electrodes, however, this is not possible. When measuring the capacitance corresponding to the outermost electrode, a collective electrode is not necessarily formed of several electrodes. The formed capacitances of the sensor can be measured by changing the one electrode connected to the first potential. If a collective electrode is formed of several electrodes by connecting them electrically together, said one electrode is advantageously left between two such electrodes that belong to a collective electrode. Advantageously, a total of five capacitances are measured, one capacitance corresponding to each electrode.

In an embodiment, several electrodes are provided. Thus, one electrode can be connected to a first potential and two electrodes to a second potential. The remaining electrodes can be, one by one, either connected to said second potential, or electrically insulated from both the first and the second potential. The formed capacitance of the sensor can be measured. The formed capacitances of the sensor can be measured by changing the one electrode connected to the first potential. If the electrodes are placed in parallel, said one electrode advantageously remains between said two electrodes. At the edge of a row of electrodes, however, this is not possible. If a collective electrode is formed of several electrodes by connecting them electrically together, said one electrode is advantageously left between two such electrodes that belong to a collective electrode. Advantageously, the capacitance of each electrode is measured with respect to at least one other electrode. Advantageously, the capacitance of at least one electrode is measured with respect to one collective electrode, said electrode being electrically insulated from said collective electrode, and said collective electrode being formed by connecting at least two electrodes electrically together.

Consequently, for example the following has been presented above:
Example A1. A sensor (100) comprising
   - a frame (110);
   - a first conductive electrode (120) provided in and/or on a frame (110),
   - a second conductive electrode (121) provided in and/or on a frame (110), and
   - a third conductive electrode (122) provided in and/or on a frame (110), in which sensor (100)
   - each of the three electrodes (120, 121, 122) is electrically insulated from the other electrodes, wherein capacitances of the sensor are formed between each of the three electrodes and at least one other electrode,
characterized in that
   - said frame (110) comprises textile or fabric, and
   - said electrodes (120, 121,122) are aligned in parallel in the frame (110).

Example A2. The sensor (100) according to example A1, characterized in that the electrodes (120, 121, 122) are provided in a plane, wherein the capacitance of the sensor is configured to change in such a way that the spacing between the electrodes remains substantially constant.

Example A3. The sensor according to example A1 or A2, characterized in that the first or second conductive electrode comprises one of the following:
- electroconductive elastomer,
- electroconductive ink,
- thread treated to be electroconductive,
- a band formed of electroconductive fabric, and
- an electric wire.

Example A4. The sensor (100) according to any of the examples A1 to A4, characterized in that
- the conductive electrodes (120, 121, 122) are in the form of continuous conductors, and
- the distance of a conductive electrode (120, 121, 122) from the closest other conductive electrode is in the range of 0.5 to 25 cm.

Example A5. The sensor according to example A4, characterized in that
- the frame (110) has a planar shape,
- the dimension of the frame (110) in a first direction is between 80 cm and 250 cm, and
- the dimension of the frame (110) in a second direction transverse to the first direction is between 60 cm and 250 cm, wherein the sensor is available for measuring the movement of a person in a lying position.

Example A6. The sensor according to example A5, characterized in that
- the electrodes extend in said first direction of the frame (110), and
- the length of the electrodes in said first direction is at least 30 cm.

Example A7. The sensor (100) according to any of the examples A4 to A6, characterized in that
- the frame (110) has a planar shape, wherein the frame has three dimensions,
- the frame (110) has a thickness corresponding to the smallest one of said dimensions,
- the electrodes are arranged in the top part of the frame (110) or on the frame (110), in the thickness direction of the frame, wherein the measuring sensitivity of the sensor is good.

Example A8. A mattress topper, a mattress, or a bed sheet comprising a sensor according to any of the examples PA1 to PA7.

Example A9. A mattress topper, a mattress, or a bed sheet according to example A8, further comprising a measuring unit (460) configured to measure the capacitances of the sensor.

Example A10. A method for measuring the movement of a human body, the method comprising
- providing a sensor comprising at least three conductive electrodes (120, 121, 122),
- electrically insulating the first conductive electrode (120) of the sensor from at least one other electrode (121, 122) of the sensor, wherein a first capacitance of the sensor is formed between the first conductive electrode (120) and at least one other electrode (121, 122),
- measuring said first capacitance of the sensor,
- measuring at least a second capacitance of the sensor by means of the sensor, and
- measuring the movement of a human body by means of said at least two capacitances,
characterized by
- providing a sensor according to any of the examples A1 to A7, or a mattress, a bed sheet, or a mattress topper according to example 8,
- electrically connecting the first conductive electrode (120) of the sensor and the second conductive electrode (122) of the sensor together, wherein
- a second collective electrode of the sensor is formed,
- the second electrode (121) of the sensor is electrically insulated from said second collective electrode of the sensor, wherein the second capacitance of the sensor is formed between the second conductive electrode (121) and said second collective electrode, and
- measuring said second capacitance of the sensor, wherein
- the movement of a human body is measured by means of said first capacitance of the sensor and said second capacitance of the sensor.

On the other hand, for example the following has been presented above:
Example B1. A method for measuring at least two capacitances, comprising
   - providing three conductive electrodes (120, 121, 122), wherein a sensor is formed, which comprises said three electrodes,
   - electrically insulating the first conductive electrode (120) of the sensor from at least one other electrode (121, 122) of the sensor, wherein a first capacitance of the sensor is formed between the first conductive electrode (120) and at least one other electrode (121, 122), and
   - measuring said first capacitance of the sensor,
   characterized by
   - electrically connecting the first conductive electrode (120) of the sensor and the second conductive electrode (122) of the sensor together, wherein
   - a second collective electrode of the sensor is formed,
   - the second electrode (121) of the sensor is electrically insulated from said second collective electrode of the sensor, wherein the second capacitance of the sensor is formed between the second conductive electrode (121) and said second collective electrode, and
   - measuring said second capacitance of the sensor.
Example B2. The method according to example B1, comprising
   - providing a fourth conductive electrode (123), wherein a sensor is formed, which comprises said three electrodes (120, 121, 122) and the fourth electrode (123),
   - forming a second collective electrode of the sensor by electrically connecting the first (120), the third (122), and the fourth (123) conductive electrode of the sensor together,
   - electrically insulating the second electrode (121) of the sensor from said second collective electrode of the sensor, wherein the second capacitance of the sensor is formed between the second conductive electrode (121) and the second collective electrode,
   - measuring said second capacitance of the sensor.
Example B3. The method according to example B2, comprising
   - providing further conductive electrodes, wherein a sensor is formed which comprises said electrodes,
   - forming a second collective electrode of the sensor by electrically connecting all the electrodes of the sensor together, except for the second electrode (121) of the sensor,
   - electrically insulating the second electrode (121) of the sensor from said second collective electrode of the sensor, wherein the second capacitance of the sensor is formed between the second conductive electrode (121) and the second collective electrode,
   - measuring said second capacitance of the sensor.
Example B4. The method according to example B1, comprising
   - providing a fourth conductive electrode (123), wherein a sensor is formed, which comprises said three electrodes (120, 121, 122) and the fourth electrode (123),
   - forming a second collective electrode of the sensor by electrically connecting the first (120) and the third (122) conductive electrodes of the sensor together,
   - electrically insulating the fourth electrode (123) of the sensor from said second collective electrode of the sensor,
   - electrically insulating the second electrode (121) of the sensor from said second collective electrode and said fourth electrode (123) of the sensor, wherein the second capacitance of the sensor is formed between the second conductive electrode (121) and said second collective electrode,
   - measuring said second capacitance of the sensor.
Example B5. The method according to example B4, comprising
   - providing further electrodes, wherein a sensor is formed which comprises said electrodes,
   - forming a second collective electrode of the sensor by electrically connecting the first (120) and the third (122) conductive electrodes of the sensor together,
   - electrically insulating the second electrode (121) of the sensor from said second collective electrode of the sensor,
   - electrically insulating the other electrodes from said second collective electrode of the sensor and from said second electrode (121) of the sensor, wherein the second capacitance of the sensor is formed between the second conductive electrode (121) and said second collective electrode,
   - measuring said second capacitance of the sensor.
Example B6. The method according to any of the examples B1 to B5, wherein
   - the electrodes are placed in parallel, and
   - when forming said second collective electrode of the sensor by connecting the first (120) and third (122) conductive electrodes of the sensor together, said second conductive electrode (121) of the sensor is left between two electrodes (120, 122) belonging to the collective electrode.
Example B7. The method according to any of the examples B1 to B6, comprising
   - measuring the capacitance of each electrode with respect to at least one other electrode.
Example B8. A method for measuring the movement of a human body, the method comprising
   - measuring at least two capacitances according to any of the examples B1 to B7, and
   - measuring the movement of a human body by means of said at least two capacitances.
Example B9. The method according to example B8, wherein
   - said electrodes (120, 121,122) are provided in a frame (110), wherein
   - said sensor comprises the frame (110) and said electrodes (120, 121, 122), and
   - said frame (110) comprises textile or fabric.
Example B10. The method according to example B9, wherein
   - a mattress, a mattress topper or a bed sheet is used as said frame (110).
Example B11. A device configured to measure at least two capacitances according to any of the examples B1 to B10.

The method according to any of the examples B1 to B10 can be applied, for example, by providing electrodes in a sensor or a mattress according to any of the examples A1 to A9.

According to example A9, the sensor may comprise a measuring unit 460. The measuring unit 460 may be configured to measure at least two capacitances according to any of the examples B1 to B10.

The invention is not limited to the above-presented embodiments, but it can be applied within the scope of the appended claims.

## Claims

1. A system comprising a sensor (100) comprising
- a planar frame (110);
- a first conductive electrode (120) provided in and/or on a frame (110),
- a second conductive electrode (121) provided in and/or on a frame (110), and
- a third conductive electrode (122) provided in and/or on a frame (110), in which sensor (100)
- each of the three electrodes (120, 121, 122) is electrically insulated from the other electrodes, wherein capacitances of the sensor are formed between each of the three electrodes and at least one other electrode, and
- the electrodes (120, 121, 122) are configured in such a way that a space is left between the electrodes in a direction parallel to the plane of the frame (110), wherein
- said electrodes (120, 121,122) are arranged in parallel in the frame (110),
- said frame (110) comprising textile or fabric, and
- the system further comprising a measuring unit (460),
**characterized in that**
- the measuring unit (460) is configured to measure at least one capacitance of the sensor in such a way that the capacitance of an electrode (120, 121, 122) is measured with respect to at least one adjacent electrode (121, 122, 120).

2. The system according to claim 1, **characterized in that** the sensor (100) is arranged in a mattress topper, a mattress, or a bed sheet.

3. The system according to claim 1 or 2, **characterized in that**
- the frame (110) of the sensor (100) has three dimensions, wherein
- the frame (110) has a thickness corresponding to the smallest one of said dimensions, and
- all the electrodes are arranged in the top part of the frame (110) or on the frame (110), in the thickness direction of the frame, wherein the measuring sensitivity of the sensor is good.

4. The system according to any of the claims 1 to 3, **characterized in** the electrodes (120, 121,122) of the sensor (100) are arranged in a plane.

5. The system according to any of the claims 1 to 4, **characterized in that**
- the conductive electrodes (120, 121, 122) are in the form of extending conductors, and
- the distance of a conductive electrode (120, 121, 122) from the closest other conductive electrode is in the range of from 0.5 to 25 cm.

6. The system according to any of the claims 1 to 5, **characterized in that** each electrode (120, 121, 122) of the sensor (100) can be connected by control electronics to both the first electric potential and the second electric potential, in an alternating manner.

7. The use of a sensor (100), the sensor (100) comprising
- a planar frame (110);
- a first conductive electrode (120) provided in and/or on a frame (110),
- a second conductive electrode (121) provided in and/or on a frame (110), and
- a third conductive electrode (122) provided in and/or on a frame (110), in which sensor (100)
- each of the three electrodes (120, 121, 122) is electrically insulated from the other electrodes, wherein capacitances of the sensor are formed between each of the three electrodes and at least one other electrode, and
- the electrodes (120, 121, 122) are configured in such a way that a space is left between the electrodes in a direction parallel to the plane of the frame (110), whereby
- said electrodes (120, 121, 122) are arranged in parallel in the frame (110), and
- said frame (110) comprises textile or fabric,
**characterized in that** in the use,
- the capacitance of an electrode (120, 121, 122) of the sensor (100) is measured with respect to at least one adjacent electrode (121, 122, 120).

8. A method for measuring a capacitance, wherein a sensor (100) is provided, the sensor comprising
- a planar frame (110);
- a first conductive electrode (120) provided in and/or on a frame (110),
- a second conductive electrode (121) provided in and/or on a frame (110), and
- a third conductive electrode (122) provided in and/or on a frame (110), in which sensor (100)
- each of the three electrodes (120, 121, 122) is electrically insulated from the other electrodes, wherein capacitances of the sensor are formed between each of the three electrodes and at least one other electrode, and
- the electrodes (120, 121, 122) are configured in such a way that a space is left between the electrodes in a direction parallel to the plane of the frame (110), wherein
- said electrodes (120, 121, 122) are arranged in parallel in the frame (110), and
- said frame (110) comprises textile or fabric,
**characterized in that** the method comprises
- measuring the capacitance of an electrode (120, 121, 122) of the sensor (100) with respect to at least one adjacent electrode (121, 122, 120).

9. The use according to claim 7 or the method according to claim 8, **characterized in that** the sensor (100) is arranged in a mattress topper, a mattress, or a bed sheet.

10. The use or method according to any of the claims 7 to 9, **characterized in that**
- the sensor comprises several electrodes, and
- at least two capacitances are measured by connecting one electrode to the first potential and at least one other electrode to the second potential in an alternating manner.

11. The use or method according to claim 10, **characterized in that**
- each electrode (120, 121, 122) is connected to both the first electric potential and the second electric potential in an alternating manner.

12. The use or method according to any of the claims 7 to 11, wherein
- the second conductive electrode (121) of the sensor is electrically connected to one other conductive electrode of said sensor, wherein a first collective electrode of the sensor is formed,
- the first conductive electrode (120) of the sensor is electrically insulated from said first collective electrode of the sensor, wherein a first capacitance of the sensor is formed between the first conductive electrode (120) and at least one other electrode (121, 122) of the sensor, and
- said first capacitance of the sensor is measured,
**characterized in that**
- the first conductive electrode (120) and the third conductive electrode (122) of the sensor are electrically connected together, wherein the second collective electrode of the sensor is formed,
- the second electrode (121) of the sensor is electrically insulated from said second collective electrode of the sensor, wherein the second capacitance of the sensor is formed between the second conductive electrode (121) and said second collective electrode, and
- said second capacitance of the sensor is measured.

13. The use or method according to claim 12, wherein
- the second conductive electrode (121) and the third conductive electrode (122) of the sensor are electrically connected together, wherein the first collective electrode of the sensor is formed, and
- the first conductive electrode (120) of the sensor is electrically insulated from said first collective electrode of the sensor, wherein a first capacitance of the sensor is formed between the first conductive electrode (120) and at least one other electrode (121, 122) of the sensor,
- said first capacitance of the sensor is measured,
**characterized in that**
- the first conductive electrode (120) and the third conductive electrode (122) of the sensor are electrically connected together, wherein the second collective electrode of the sensor is formed,
- the second electrode (121) of the sensor is electrically insulated from said second collective electrode of the sensor, wherein the second capacitance of the sensor is formed between the second conductive electrode (121) and the second collective electrode, and
- said second capacitance of the sensor is measured.

14. The use or method according to claim 12 or 13, **characterized in that**
- when forming said second collective electrode of the sensor by connecting the first (120) and third (122) conductive electrodes of the sensor together, said second conductive electrode (121) of the sensor is left between two electrodes (120, 122) belonging to the collective electrode.

15. Use of a sensor, or a method, for measuring the movements of a human body, **characterized in that**
- at least two capacitances are measured according to the use or method according to any of the claims 7 to 14, and
- the movements of the human body are measured by means of said at least two capacitances.
